# EUROPEAN PATENT APPLICATION

(11) **EP 2 927 686 A1**
(43) Date of publication of application: **07.10.2015**
(21) Application number: 13859129.2
(22) Date of filing: 26.11.2013
(51) Int. Cl.: G01N 33/52, G01N 33/53, G01N 37/00, G01N 21/78

(54) **AUTOMATIC IN VITRO DIAGNOSTIC APPARATUS INCLUDING INCLINED ROTATING PLATE**

(30) Priority: 27.11.2012 KR 20120135018
(71) Applicant: LG Life Sciences Ltd., Seoul 110-783 (KR)
(72) Inventor: OH, Jae Hoon, Daejeon 305-738 (KR); PARK, Moon Kyu, Daejeon 305-738 (KR); KIM, Seong Wook, Daejeon 305-738 (KR); LEE, Joon Hee, Daejeon 305-738 (KR)
(74) Representative: Gillard, Richard Edward
(86) International application number: PCT/KR2013/010761
(87) International publication number: WO 2014/084561

(57) **Abstract**

According to the present invention, an automatic *in vitro* diagnosis apparatus including an inclined rotating stirrer is an apparatus for the automatic *in vitro* diagnosis of a clinical specimen or a reagent. The present invention includes: a clinical specimen storage unit which stores a clinical specimen; a reagent storage unit which stores a reagent; a code reader unit which recognizes an identification code attached to the clinical specimen storage unit or to the reagent storage unit; a dispenser which suctions the clinical specimen and the reagent from the clinical specimen storage unit and the reagent storage unit, and transports the clinical specimen and the reagent which are suctioned; and a rotating stirrer which receives the clinical specimen and the reagent from the dispenser and rotates about an axis of rotation so as to stir the clinical specimen and the reagent. The axis of rotation of the rotating stirrer forms a predetermined angle with the direction of gravity, and a series of processes for storing, transporting, and stirring the clinical specimen and the reagent are integrated so as to maximize the efficiency of the stirring and the efficiency of the operations.

## Description

### [Technical field]

The present invention is related to an automatic *in vitro* diagnostic apparatus including an inclined rotating plate. More specifically, the present invention is related to an automatic *in vitro* diagnostic apparatus for automatic dispensing of a clinical specimen and a reagent stored in a storage unit to a rotating plate, and then the rotating plate incubates the clinical specimen and the reagent by rotating around an inclined rotating axis so as to detect the incubated clinical specimen and the reagent.

### [Background]

A reagent for detection *in vitro* means a chemical for detecting an object which is to be detected (a specific material of the human body-i.e., a protein, a nucleic acid, a sugar, a lipid, a small molecule, and so on) in a clinical specimen (a material of the human body like blood or urine and so on).

A method for detecting a specific material existing in a clinical specimen is as follows. First, a material which can react with a reaction material is fixed at a fixing supporter, and a reagent is reacted with it. The reaction material is chemically bonded with a specific material which is fixed at the fixing supporter by the reaction, and non-bonding materials in the clinical specimen can be eliminated by a washing process. Finally, by using various materials which can generate a signal, the detection material which is fixed at the fixing supporter can be identified.

For example, the method explained above is widely used for molecular diagnosis (for example, a test for the genotype of a microorganism and an infectious virus including HPV, or a test for major histocompatibility in the immune system) as well as an immunodiagnosis using an antigen-antibody reaction like tests for allergic diseases, test for autoantibody or a test for immunity from infectious diseases.

However, in the traditional methods for detection, a process for inserting the clinical specimen and the reagent into a reaction container for a while and washing the container is usually repeated in order to detect the object to be detected. In the process explained above, to promote the reaction, a process of incubating can be implemented added during the reaction time.

A series of incubating and washing processes calls for too much labor, and it could be affected by workers' abilities and experiences, so various automation equipment is used to overcome these problems.

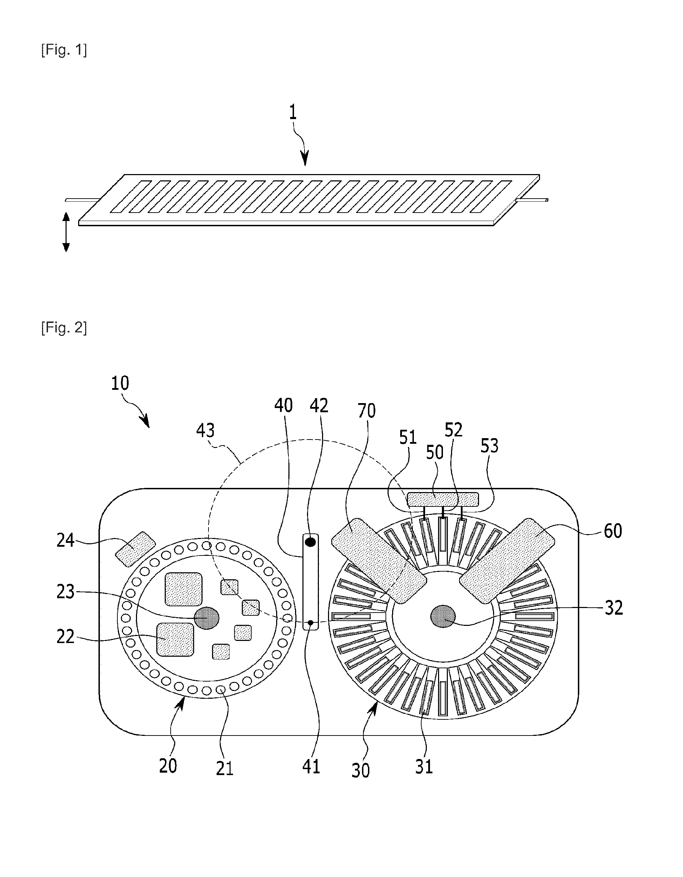

Fig. 1 is schematic diagram of an existing stirrer which incubates a clinical specimen and a reagent by moving upward and downward.

Until now, in order to invent automation equipment for detecting a clinical specimen *in vitro* using membrane or slide glass as a fixing supporter, the stirrer shown in Fig. 1 has been widely used in reacting the clinical specimen with the reagent.

The traditional rocking shaker as shown in Fig. 1 is an apparatus wherein the reaction container is parallelized in a rectangle plate in a straight line and incubates upward and downward about an axis.

In the rocking shaker method, the stored contents are repeatedly moved upward and downward along the gravity direction, so it has an effect wherein the clinical specimen and the reagent are mixed. However, this method has a problem in that the automation apparatus could not be easily made because a pipet had to be moved along a vertical line and a horizontal line in order to dispense the clinical specimen and the reagent, thus making the apparatus complex. Furthermore, the traffic line of the pipet is long so it has a problem in that the total time for deducing the result is delayed.

### [Technical Problem]

The present invention according to an automatic *in vitro* diagnostic apparatus including an inclined rotating plate is designed to solve the technical problems explained above and has various purposes as follows.

First, the present invention is directed to consolidate a series of processes which include storing, dispensing and incubating a clinical specimen and a reagent.

Second, the present invention is directed to reduce the complexity and inefficiency in accordance with the usage of the existing rocking shaker.

The objects of the present invention are not limited to the above, and other objects would be clearly understood by a skilled artisan from the following disclosure.

### [Technical Solution]

In this regard, the present invention according to an automatic *in vitro* diagnostic apparatus including an inclined rotating plate provides the following solutions.

The present invention is related to an apparatus for automatically diagnosing a clinical specimen or a reagent *in vitro,* and the apparatus of the present invention comprises: a clinical specimen storage unit which accommodates and stores the clinical specimen, and attaches a code having information of the accommodated and stored clinical specimen to a surface thereof; a reagent storage unit which accommodates and stores the reagent, and attaches a code having information of the accommodated and stored clinical specimen to a surface thereof; a code reader unit which recognizes an identification code attached to the clinical specimen storage unit or to the reagent storage unit; a dispenser which receives the information of the clinical specimen and the information of the reagent from the code reader unit, recognizes the clinical specimen storage unit and the reagent storage unit storing the information of the clinical specimen and the information of the reagent to be taken, takes the clinical specimen and the reagent to be taken, and transports the clinical specimen and the reagent which are taken; and a rotating plate which receives the clinical specimen and the reagent from the dispenser and rotates about a rotating axis so as to incubate the clinical specimen and the reagent, wherein the rotating axis of the rotating plate has a predetermined angle against the gravity direction.

The dispenser of the present invention comprises: a rotating bar which rotates with one part fixed at the rotating axis; and a probe which is positioned at another part of the rotating bar, takes the clinical specimen and the reagent, and dispenses them.

The present invention comprises: a material plate unit which accommodates the clinical specimen storage unit and the reagent storage unit, and rotates about a rotating axis, wherein the material plate unit stops rotating, if the code reader unit recognizes the identification code of the clinical specimen and the reagent which are to be diagnosed by rotating, and then the dispenser takes the clinical specimen of the clinical specimen storage unit having the recognized identification code and the reagent of the reagent storage unit having the recognized identification code, and transports the taken clinical specimen and the taken reagent to the rotating plate.

The present invention further comprises: a suction unit which eliminates residues of the clinical specimen and the reagent from the rotating stirrer by suctioning the residues of the clinical specimen and the reagent.

The present invention further comprises: a detecting unit which detects the clinical specimen and the reagent which are incubated by the rotating plate.

The present invention further comprises: a drying unit which sends air to the rotating plate so as to dehydrate it after the suction unit suctions the residues of the clinical specimen and the reagent of the rotating plate.

The present invention further comprises: a vibrator which is positioned at the rotating axis and applies the rotating plate with upward and downward movements.

### [Advantageous Effects]

The present invention according to an automatic *in vitro* diagnostic apparatus including inclined rotating plate has the following various effects.

First, the present invention improves the efficiency by consolidating the processes of storing, dispensing and incubating a clinical specimen and a reagent.

Second, the present invention improves the performances of incubating by forming a rotating axis which has a predetermined angle against the gravity direction.

The effects of the present invention are not limited to the above, and other effects would be clearly understood by a skilled artisan from the following disclosure.

### [Description of Drawings]

Fig. 1 is a schematic diagram of an existing stirrer which incubates a clinical specimen and a reagent by moving upward and downward.
Fig. 2 is a plane figure which shows each element of the present invention according an automatic *in vitro* diagnostic apparatus including an inclined rotating plate.
Fig. 3 is a side view of the inclined rotating plate in accordance with an preferred embodiment of the present invention.

### [Detailed Description]

The present invention according to an automatic *in vitro* diagnostic apparatus including an inclined rotating plate may have various embodiments so it discloses specific embodiments with drawings and provides detailed explanations. However, it does not limit the scope of the present invention to the specific embodiments, so it must be understood that the present invention comprises all alterations, equivalents or alternatives which are included within the technical scope or technical idea of the present invention.

Hereinafter, an automatic *in vitro* diagnostic apparatus including inclined rotating stirrer in accordance with the present invention will be described in detail.

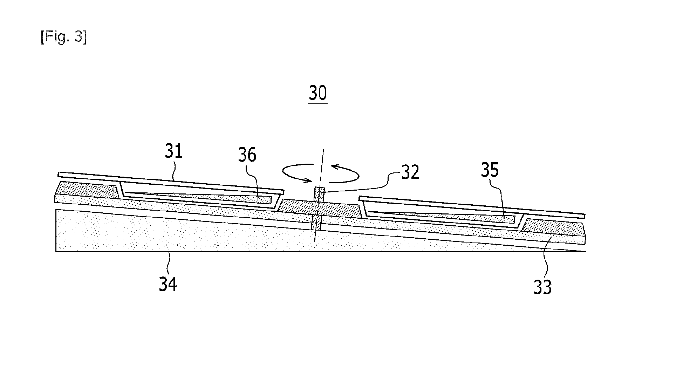

Fig. 2 is a plane figure which shows each element of the present invention according to an automatic *in vitro* diagnostic apparatus including an inclined rotating plate. Fig. 3 is a side view of the inclined rotating plate in accordance with a preferred embodiment of the present invention.

The automatic *in vitro* diagnostic apparatus including inclined rotating plate in accordance with the present invention is directed to an apparatus for automatically diagnosing the clinical specimen and the reagent *in vitro* so it comprises a clinical specimen storage unit 21; a reagent storage unit 22; a code reader unit 24; a dispenser 40; and a rotating plate 30.

As shown in Fig. 2, the clinical specimen storage unit 21 can be equipped with a tube including the clinical specimen. Hereinafter, the clinical specimen means materials of the human body like blood or urine.

As shown in Fig. 2, the reagent storage unit 22 can be equipped with various types of containers including reagents, and the reagents physically or chemically react with the clinical specimen and facilitate the diagnosing works so as to diagnose the specific materials from the human body-i.e., a protein, a nucleic acid, a sugar, a lipid or small molecules.

As shown in Fig. 2, the clinical specimen storage unit 21 and the reagent storage unit 22 can be combined and accommodated in one container like a material plate unit 20, which will be explained below, or separated and accommodated in two separate containers, respectively.

The clinical specimen stored in the clinical specimen storage unit 21 and the reagent stored in the reagent storage unit 22 are drawn respectively, and each of them should be transported to the rotating plate respectively. After that, the dispenser 40 draws the clinical specimen and the reagent from the clinical specimen storage unit 21 and the reagent unit 22, and transports and dispenses them to the rotating plate 30.

The dispenser 40 is inputted information about the types of clinical specimens to be drawn and the types of reagents to be drawn in accordance with pre-entered information. In addition, the information about the mass of the clinical specimen and the reagent are inputted into the dispenser 40 so that an adequate amount of the clinical specimen and the reagent is drawn and dispensed.

After that, the dispenser 40 transports the drawn clinical specimen and the drawn reagent to the rotating plate 30, and the rotating plate 30 rotates about a rotating axis 32 and incubates them. In this case, as shown in Fig. 3, the rotating axis 32 has a predetermined angle against the gravity direction, so the rotating plane formed by the rotating plate 30 also has a predetermined angle against the horizontal angle.

A code-for example, a bar code or a QR code, etc. -is stuck on the clinical specimen unit 21, and the code includes information about the clinical specimen stored in the clinical specimen unit 21. Likewise, a code-for example, a bar code or a QR code, etc. -is stuck on the reagent storage unit 22, and the code includes information about the reagent stored in the reagent storage unit 22.

The code reader unit 24 recognizes the code stuck on the clinical specimen storage unit 21 and the reagent storage unit 22, and recognizes the type of clinical specimen and the reagent stored in the clinical specimen storage unit 21 and the reagent storage unit 22. After that, the code reader unit 24 transports the information to the dispenser 40.

If the rotating axis 32 of the rotating plate 30 is the same as the direction of gravity, the centrifugal force resulting from the turning force of the rotating plate 30 is uniformly generated, so the incubating efficiency decreases. Thus, if the rotating axis 32 has a predetermined angle against the gravity direction and rotates, as shown in Fig. 3, the force of gravity applied to the clinical specimen and the reagent is reinforced and weakened alternately, so the incubating efficiency can be increased.

Hereinafter, the predetermined angle is not limited to a specific value, so it could be properly adopted by a skilled artisan in accordance with the turning torque or the performance of the rotating plate 30 and the types of the clinical specimen and the reagent.

As shown in Fig. 2, a plurality of reaction container 31 are placed at the rotating plate 30, and the stored clinical specimen and the stored reagent are incubated.

As shown in Fig. 2, the reaction container 31 comprises a fixing member like a plastic, a membrane or a slide glass, and a material which reacts on the clinical specimen can be spread on the fixing member.

As shown in Fig. 2, the dispenser 40 can comprise the rotating bar and probe 41. As shown in Fig. 2, one end of the rotating bar rotates about the rotating axis and the other end of the rotating bar forms a circular trajectory 43.

The probe 41 is placed at the other end of the rotating bar, forms a circular trajectory 43, draws the clinical specimen and the reagent from the clinical specimen storage unit 21 and the reagent storage unit 22 respectively, and then dispenses them into the rotating plate 30.

The probe 41 is connected to a pump by a proper tube, and is constituted to draw and dispense an adequate amount of the clinical specimen and the reagent.

As explained above, the material plate unit 20 can comprise the clinical specimen storage unit 21 and the reagent storage unit 22, and the material plate unit 20 rotates about the rotating axis 23.

While the material plate unit 20 rotates, the code reader unit 24 recognizes the code which is stuck on the clinical specimen storage unit 21 and the reagent storage unit 22. If the object accommodating the clinical specimen and the reagent to be detected is recognized, the material plate unit 20 stops rotating.

After that, the dispenser 40 moves to the clinical specimen and the reagent of purpose. In other words, as explained above, the probe 41 moves with the rotating bar rotating about the rotating axis 42, draws the clinical specimen and the reagent of purpose and then transports them to the rotating plate 30.

As shown in Fig. 2, the present invention according to an automatic *in vitro* diagnostic apparatus including an inclined rotating plate can further comprise a suction unit 50.

The suction unit 50 draws the residues of the clinical specimen and the reagent from the rotating plate 30 (more specifically, from the reaction container 31) by using straws 51, 52, 53, and then cleans the reaction container 31.

In addition, if a process for drying is needed after the reaction and the cleaning in order to facilitate measurement, a drying unit 70 removes the remaining moisture by applying (warm) wind. While the rotating plate 30 rotates with uniform speed, the drying unit 70 operates, and then all of the reaction containers are dried.

The detecting unit 60 detects the target material from the reaction container of the rotating plate 30 and measures it. The detecting unit 60 is constituted to detect a color, a light, etc., in accordance with the principle of the detection. The rotating plate 30 transports each transaction container to a position where the detecting unit 60 detects them by rotating, and then the detecting unit 60 sequentially detects them.

The present invention according to an automatic *in vitro* diagnostic apparatus including an inclined rotating plate can further comprise a vibrator (not shown). The vibrator is attached to the rotating axis 32 of the rotating plate 30 and applies the rotating plate 30 with upward and downward movements.

As explained above, since the vibrator causes vibrations to the rotating plate 30, it complements the performance of the inclined rotating plate 30.

The singular forms used in the specification are intended to include the plural forms as well, unless the context clearly indicates otherwise. It will be further understood that the terms "comprises" and/or "comprising," or "includes" and/or "including" specify the presence of stated features, regions, integers, steps, operations, elements, and/or components, but do not preclude the presence or addition of one or more other features, regions, integers, steps, operations, elements, components, and/or groups thereof.

The scope of the present disclosure is defined by the appended claims, and parentheses used in the claims are not intended to limit the invention but to clearly express the relevant elements. The limitations within the parentheses should also be interpreted as essential elements.

### Reference symbols

10: automatic *in vitro* diagnostic apparatus
20: material plate unit
21: clinical specimen storage unit
22: reagent storage unit
23: rotating axis of material plate unit
24: code reader unit
30: rotating plate
31: reaction container
32: rotating axis of rotating plate
40: dispenser
41: probe
42: rotating axis of rotating bar
43: trajectory of probe
50: suction unit
51, 52, 53: suction straw
60: detecting unit
70: drying unit

## Claims

1. An apparatus for automatically diagnosing a clinical specimen or a reagent *in vitro,* the apparatus comprising:
a clinical specimen storage unit which accommodates and stores the clinical specimen, and attaches a code having information of the accommodated and stored clinical specimen to a surface thereof;
a reagent storage unit which accommodates and stores the reagent, and attaches a code having information of the accommodated and stored clinical specimen to a surface thereof;
a code reader unit which recognizes an identification code attached to the clinical specimen storage unit or to the reagent storage unit;
a dispenser which receives the information of the clinical specimen and the information of the reagent from the code reader unit, recognizes the clinical specimen storage unit and the reagent storage unit storing the information of the clinical specimen and the information of the reagent to be taken, takes the clinical specimen and the reagent to be taken, and transports the clinical specimen and the reagent which are taken; and
a rotating plate which receives the clinical specimen and the reagent from the dispenser and rotates about a rotating axis so as to incubate the clinical specimen and the reagent,
wherein the rotation axis of the rotating plate has a predetermined angle against the gravity direction.

2. The apparatus according to Claim 1, wherein the dispenser comprises:
a rotating bar which rotates with one part fixed at the rotating axis; and
a probe which is positioned at another part of the rotating bar, takes the clinical specimen and the reagent, and dispenses them.

3. The apparatus according to Claim 2, further comprising:
a material plate unit which accommodates the clinical specimen storage unit and the reagent storage unit, and rotates about a rotating axis,
wherein the material plate unit stops rotating, if the code reader unit recognizes the identification code of the clinical specimen and the reagent which are to be diagnosed by rotating, and then the dispenser takes the clinical specimen of the clinical specimen storage unit having the recognized identification code and the reagent of the reagent storage unit having the recognized identification code, and transports the taken clinical specimen and the taken reagent to the rotating plate.

4. The apparatus according to Claim 1, further comprising:
a suction unit which eliminates residues of the clinical specimen and the reagent from the rotating plate by suctioning the residues of the clinical specimen and the reagent.

5. The apparatus according to Claim 1, further comprising:
a detecting unit which detects the clinical specimen and the reagent which are incubated by the rotating plate.

6. The apparatus according to Claim 4, further comprising:
a drying unit which sends air to the rotating plate so as to dehydrate it after the suction unit suctions the residues of the clinical specimen and the reagent of the rotating plate.

7. The apparatus according to Claim 1, further comprising:
a vibrator which is positioned at the rotation axis and applies the rotating plate with upward and downward movements.
